# EUROPÄISCHE PATENTSCHRIFT

(11) **EP 3 911 378 B1**
(45) Veröffentlichungstag und Bekanntmachung des Hinweises auf die Patenterteilung: **25.09.2024**
(21) Anmeldenummer: 19809626.5
(22) Anmeldetag: 19.11.2019
(51) Int. Cl.: A61M 5/142

(54) **VERBESSERTES MODULARES VERABREICHUNGSGERÄT**
IMPROVED MODULAR ADMINISTRATION APPLIANCE
APPAREIL D'ADMINISTRATION MODULAIRE AMÉLIORÉ

(30) Priorität: 17.01.2019 CH 552019
(43) Veröffentlichungstag der Anmeldung: 24.11.2021
(73) Patentinhaber: TecMed AG, 3400 Burgdorf (CH)
(72) Erfinder: STREIT, Ursina, 3322 Schönbühl (CH); BURI, Thomas, 3400 Burgdorf (CH); HOFER, Christophe, 3400 Burgdorf (CH); HOSTETTLER, Patrick, 3415 Hasle (CH); BIGLER, Bernhard, 3362 Niederönz (CH); BOSSHARD, Simon Martin, 3006 Bern (CH); STECK, Jürg, 3422 Kirchberg (CH); LEUZINGER, Thomas, 3110 Münsingen (CH); ZÜRCHER, Clemens, 3439 Ranflüh (CH)
(74) Vertreter: Meier Obertüfer, Jürg
(86) Internationale Anmeldenummer: PCT/IB2019/059913
(87) Internationale Veröffentlichungsnummer: WO 2020/148581

(56) Entgegenhaltungen:
- US-A1- 2015 025 503
- US-A1- 2016 339 174
- US-A1- 2018 264 189
- US-A1- 2018 264 192

## Beschreibung

### TECHNISCHES GEBIET

Die vorliegende Erfindung betrifft das Gebiet der Verabreichungsgeräte, insbesondere der tragbaren Infusionspumpen, speziell auf der Haut von benutzenden Personen trag- und befestigbaren Infusionspumpen für insbesondere Insulin.

### HINTERGRUND DER ERFINDUNG

Aus dem Stand der Technik sind Verabreichungsgeräte bekannt, welche zur Verabreichung von fluiden Medikamentenformulierungen verwendet werden können. Aus der US 20040064096 A1 ist eine Verabreichungsvorrichtung zur Verabreichung von Insulin bekannt, welche direkt auf der Haut der benutzenden Person fixiert werden kann. Es handelt sich dabei um eine sogenannte Pflasterpumpe, wobei auch der Begriff Patch Pumpe im Deutschen dafür verwendet wird. Diese Pflasterpumpen haben die Vorteile, dass sie diskret unter der Kleidung getragen werden können, sehr kompakt gebaut werden können, kabellos über eine Funkfernsteuerung bedient werden, und zumeist kein separates Infusionsset benötigen. Nachteilig an der in US 20040064096 A1 ist, dass die ganze Pumpe nach Entleeren des Reservoirs entsorgt werden muss, inklusive aller potentiell wiederverwendbaren Teile.

Aus der US 2011160652 A1 ist eine Pflasterpumpe bekannt, welche modular aufgebaut ist, dabei besteht die modulare Pflasterpumpe aus einer Grundplatte, welche auf die Haut der benutzenden Person geklebt ist und durch welche eine Kanüle ins Gewebe ragt, durch welche Medikament verabreicht werden kann. Weiter umfasst die Pflasterpumpe ein Reservoirmodul, welches als Einwegmodul ausgelegt ist sowie ein verwendbares Pumpmodul. Dabei enthält das Pumpmodul u. a. die Steuerelektronik und den elektrischen Antrieb. Zur Verwendung werden Reservoirmodul und Pumpmodul zusammengesteckt. Das Reservoirmodul enthält eine Energiequelle, welche die Elektronik und den Antrieb mit Energie versorgen kann, wenn die beiden Module zusammengesteckt sind. Dazu umfassen das Reservoirmodul und das Pumpmodul Kontakte, welche beim Zusammenstecken der Module mit einander verbunden werden und so die Energieversorgung des Pumpmoduls ermöglichen. Aufgrund der einfachen Steckverbindung zwischen Reservoirmodul und Pumpmodul ist es bei der in der US 2011160652 A1 beschriebenen Vorrichtung aufwendig, eine saubere und reproduzierbare elektrische Kontaktierung zwischen Reservoirmodul und Pumpmodul herzustellen. Zum Beispiel unterliegt das Pumpmodul, welches regelmässig ausgewechselt werden muss (typischerweise alle drei Tage) und in grossen Stückzahlen hergestellt wird, geometrischen Produktionstoleranzen, welche die Kontaktierung negativ beeinflussen können. Je nach gewählter Aufteilung der Teile für den Antrieb zwischen Pumpmodul und Reservoirmodul kann es sein, dass die Steckverbindung die Antriebskräfte aufnehmen können muss, was das Design der Steckverbindung anspruchsvoller macht, resp. die Möglichkeit zur Aufteilung zwischen Pumpmodul und Reservoirmodul einschränkt. So scheint es nicht grundlos zu sein, dass in der US 2011160652 A1 die Kolbenstange im Reservoirmodul angeordnet ist und nur eine Drehbewegung vom Pumpmodul auf die Kolbenstange übertragen wird.

Ein Ansatz zur Adressierung dieser Probleme ist das lineare Zusammenstecken und Verschnappen von Modulen. US2015/025503A1 (Gary Searle et al, 2013), US2018/264192A1 (Yodfat Ofer, 2018) sowie US2016/339174A1 (Julian Shapley et al, 2016) sind Beispiele von mehrteiligen Pflasterpumpen, in welchen das Zusammenfügen von Modulen linear gelöst ist. Wie erwähnt bringen linear steckbare und wieder lösbare Verbindungen vermehrt Toleranzprobleme, was sich auf die Sicherheit und Zuverlässigkeit der Pumpe auswirken kann.

Weniger Toleranzprobleme gibt es wenn die Module mit einer Rotationsverbindung zusammengekoppelt werden. Eine solche ist zum Beispiel in US2018/264189A1 beschrieben. Dabei werden Pumpmodul und Reservoir-modul entlang einer Achse zusammengeführt und anschliessend durch Drehung um die Verbindungsachse lösbar verriegelt. Rund um die Rotationsachse sind mechanische Elemente angeordnet, die bei der Drehung ineinander greifen und so einen sicheren Halt geben, der auch grössere Kräfte übertragen kann. Diese Verbindungsart bietet alle Vorteile einer zuverlässigen und problemlos lösbaren mechanischen Verbindung, ist allerdings schwieriger zu realisieren wenn an der gleichen Schnittstelle auch elektrische Signale übertragen werden sollen.

Es besteht also ein Bedürfnis, ein modulares Verabreichungsgerät für flüssige Medikamente bereitzustellen, bei welchem ein Pumpenmodul und ein Reservoirmodul über einen Rotationsverschluss sowohl mechanisch, als auch elektrisch so miteinander verbunden werden können, dass eine sichere und zuverlässige Verabreichung des Medikamentes gewährleistet ist.

Die Begriffe "Medikament", "fluide Medikamentenformulierung" oder "medizinische Substanz" umfassen in diesem Zusammenhang jede fliessfähige medizinische Formulierung, welche geeignet ist zur kontrollierten Verabreichung mittels einer Kanüle oder Hohlnadel, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Die Angabe "distal" bezieht sich auf eine Richtung zum einstechseitigen Ende der Verabreichungsvorrichtung hin. Demgegenüber bezieht sich die Angabe "proximal" auf eine Richtung zum hinteren, dem einstechseitigen Ende gegenüberliegenden Ende der Verabreichungsvorrichtung hin.

### DARSTELLUNG DER ERFINDUNG

Es ist eine Aufgabe der Erfindung, eine modulare Verabreichungsvorrichtung bereitzustellen, bei welcher eine verbesserte elektrische Kontaktierung zwischen Modulen der Verabreichungsvorrichtung verwirklicht ist.

Die Aufgabe wird gelöst durch die Lehre des unabhängigen Anspruchs 1. Vorteilhafte Weiterentwicklungen werden in den abhängigen Ansprüchen sowie in der Beschreibung und den Figuren dargelegt.

In einer Ausgestaltung umfasst die Erfindung eine als Pflasterpumpe ausgebildete Verabreichungsvorrichtung. Die Pflasterpumpe ist dabei modular ausgebildet und umfasst ein Reservoirmodul und ein Pumpmodul. Das Reservoirmodul umfasst ein Gehäuse und ein befüllbares, von Form und Ausgestaltung her karpulenartiges Reservoir, welches durch seine zylinderartige Form eine erste Achse definiert, wobei der Querschnitt des Reservoirs rund, oval oder auch eckig sein kann und wobei die Wandungen des Reservoirs steif oder verformbar sein können. Im Reservoir ist ein verschiebbarer Stopfen angeordnet, wobei durch ein Verschieben des Stopfens das Volumen innerhalb des Reservoirs variiert, insbesondere verkleinert, werden kann. Das proximale Ende des Reservoirs ist offen und durch eine Öffnung im Gehäuse des Reservoirmoduls zugänglich, wodurch auch der Stopfen zugänglich gemacht wird. Weiter umfasst das Reservoirmodul eine erste Verbindungstruktur, welche der weiter unten beschriebenen Verbindung von Reservoirmodul und Pumpmodul dient. Die Verbindungsstruktur ist dabei um die beschriebene Gehäuseöffnung am Gehäuse des Reservoirmoduls herum angeordnet. Das Reservoirmodul umfasst weiter eine erste Kontaktanordnung, welche der elektrischen Kontaktierung mit dem Pumpmodul dient und welche vorzugsweise am Gehäuse des Reservoirmoduls angeordnet ist. In einer vorteilhaften Variante umfasst das Reservoirmodul weiter eine Energiequelle, insbesondere eine Primärzelle, zur Versorgung der Verabreichungsvorrichtung mit elektrischer Energie.

Alternativ kann das Reservoir auch als länglicher Beutel ausgestaltet sein, wobei statt eines Stopfens mindestens ein bewegliches Press- oder Quetschelement durch Zusammenpressen des Beutels für eine Verkleinerung des Reservoirvolumens sorgen könnte.

Das Pumpmodul umfasst ein Gehäuse und eine Kolbenstange, welche beweglich und zumindest teilweise im Gehäuse des Pumpmoduls gelagert ist. Das Gehäuse des Pumpmoduls weist eine Öffnung auf, durch welche die Kolbenstange bewegt werden kann. Die Kolbenstange definiert eine zweite Achse. Um die Öffnung im Pumpmodulgehäuse herum ist eine zweite Verbindungsstruktur angeordnet, welche der weiter unten beschriebenen Verbindung mit dem Reservoirmodul dient. Das Pumpmodul umfasst weiter eine zweite Kontaktanordnung, welche der elektrischen Kontaktierung mit dem Reservoirmodul dient und welche vorzugsweise am Gehäuse des Pumpmoduls angeordnet ist. In einer vorteilhaften Variante umfasst das Pumpmodul weiter eine sekundäre Energiequelle und/oder-speicher, insbesondere eine Sekundarzelle oder eine Kapazität, welche elektrische Energie aufnehmen und abgeben kann.

Reservoirmodul und Pumpmodul werden über einen dem Fachmann hinlänglich bekannten Steck-/Drehverschluss, insbesondere Bajonettverschluss, miteinander verbunden. Hierbei kann die erste Verbindungsstruktur den ersten Teil des Bajonettverschlusses darstellen und die zweite Verbindungsstruktur kann den zweiten Teil des Bajonettverschlusses darstellen. Zum Schliessen des Bajonettverschlusses werden Pumpmodul und Reservoirmodul so zueinander ausgerichtet, dass Öffnung gegen Öffnung steht und dass die erste und die zweite Achse aufeinander zu liegen kommen, es wird eine gemeinsame Achse, insbesondere eine Rotationsachse, ausgebildet. Anschliessend werden die beiden Module zusammengesteckt, wobei darauf zu achten ist, dass die Module rotativ so ausgerichtet sind, dass die Verbindungsstrukturen ein Zusammenstecken zulassen. Nach dem die Module zusammengesteckt sind, wird der Verschluss durch relatives Rotieren der Module zueinander definitiv geschlossen. Beim Rotieren werden einerseits die Module noch ein bisschen zueinander hin gezogen, so dass allfällige Produktionstoleranzen (und/oder Spiel zwischen den Teilen) ausgeglichen werden können, andererseits findet zum Schluss der Rotation eine lösbare Verriegelung statt, so dass die beiden Module nicht mehr relativ zueinander bewegt werden können. Nachdem die beiden Module miteinander verriegelt wurden, kann die Kolbenstange in das Reservoir hineinbewegt werden, so dass der Stopfen durch die Kolbenstange in distale Richtung bewegt werden kann, um Medikamentenformulierung aus dem Reservoir zu verdrängen.

Während der Rotation von Reservoirmodul und Pumpmodul relativ zueinander beim Schliessen der Bajonettverbindung wird auch der (elektrisch leitende) Kontakt zwischen der ersten Kontaktanordnung und der zweiten Kontaktanordnung hergestellt. Hierbei kann es eine Reihenfolge geben, in welcher die Kontakte der Kontaktanordnungen kontaktieren; so kann es vorteilhaft sein, wenn zuerst der Massekontakt zwischen den Modulen hergestellt wird. In einem erfindungsgemässen Beispiel ist die erste Kontaktanordnung auf derselben Gehäuseseite angeordnet wie die Öffnung im Reservoirmodul und die zweite Kontaktanordnung auf derselben Gehäuseseite wie die Öffnung im Pumpmodul.

Erfindungsgemäss haben die erste und die zweite Kontaktanordnung einen Abstand zur ersten respektive zweiten Achse (nach dem Zusammenstecken der Bildung einer gemeinsamen Rotationsachse), der grösser ist als derjenige von der ersten respektive zweiten Verbindungstruktur zur jeweiligen Achse. Das hat den Vorteil, dass der beim Schliessen der Bajonettverbindung (Rotation) beschriebene Kreisbogen von erster und zweiter Kontaktanordnung relativ zueinander länger sein kann als derjenige von erster und zweiter Verbindungsstruktur zueinander. Es entsteht dadurch eine Übersetzung des Weges, welche bei der Herstellung des elektrischen Kontaktes zwischen den Modulen einen sicheren und übergangswiderstandsarmen Kontakt ermöglicht. Die erfindungsgemässe Anordnung der ersten und zweiten Kontaktanordnung hebt sich dabei von Bajonettverschlusslösungen ab, bei welchen die Kontaktanordnungen zum Beispiel an den Verbindungstrukturen selber oder noch näher an den Achsen zu liegen kommen, weil dort der mögliche Weg zur Herstellung einer sauberen elektrischen Kontaktierung erheblich kürzer ist.

In einer Ausführung der Erfindung umfasst die erste Kontaktanordnung mindestens eine Buchse, wobei jede Buchse zum Beispiel für einen Kontakt in der Kontaktanordnung genutzt wird. Komplementär umfasst die zweite Kontaktanordnung in einer Ausführung der Erfindung mindestens einen Stift, insbesondere mindestens einen federgelagerten Stift. Beim Schliessen der Bajonettverbindung, wenn die beiden Module gegeneinander verdreht werden, beschreibt der mindestens eine Stift den Weg eines Kreisbogensegmentes und wird in die mindestens eine Buchse eingesteckt. Hierbei ist anzumerken, dass der mindestens eine Stift insbesondere tangential zum Kreisbogen angeordnet ist. Der erfindungsgemässe Vorteil entsteht nun durch die Übersetzung des Weges, da Stift(e) und Buchse(n) vom eigentlichen Bajonettverschluss abgesetzt sind.

In einer Ausführungsform der Erfindung kann die erste Kontaktanordnung als Pfostenbuchse ausgelegt sein und die zweite Kontaktanordnung als Pfostenstecker. Alternativ auch umgekehrt.

In einer vorteilhaften Ausgestaltung umfasst die erste Kontaktanordnung vier Buchsen, welche zum Beispiel zu einer einreihigen oder mehrreihigen Pfostenbuchse zusammengefasst sind, komplementär dazu umfasst die zweite Kontaktanordnung vier Kontaktstifte, welche zu einem Pfostenstecker zusammengefasst. Hierbei dienen zwei Kontakte der Energieversorgung, bei welcher elektrische Energie vom Reservoirmodul auf das Pumpmodul übertragen wird. Die beiden weiteren können sodann insbesondere zur Signalübertragung verwendet werden, insbesondere zur Übertragung von Informationen vom Reservoirmodul auf das Pumpmodul und zur Übertragung von Steuerungssignalen vom Pumpmodul auf das Reservoirmodul. In alternativen Ausgestaltung können jedoch mehr oder weniger Kontakte für die Signalübertragung vorgesehen sein.

In einer weiteren Ausgestaltung kann die erste Kontaktanordnung als einzelne Buchse mit mehreren Kontakten ausgeführt sein und entsprechend die zweite Kontaktanordnung als einzelner Stecker mit mehreren Kontakten. Dies in Analogie Verbindungstypen wie USB-, Lightning- , Headset- oder Kopfhörerverbindungen.

In einer Ausführung der Erfindung ist die erste Kontaktanordnung schwimmend am oder im Reservoirmodul gelagert. Mit dem Begriff ,schwimmend` ist gemeint, dass die erste Kontaktanordnung relativ zum Gehäuse des Reservoirmoduls eine gewisse Beweglichkeit aufweist. Dabei ist eine Beweglichkeit insbesondere (aber nicht zwingend ausschliesslich) quer zur Schliessbewegung beim Rotieren gemeint. Diese Beweglichkeit erlaubt vorteilhaft den Ausgleich von Fertigungstoleranzen am Reservoirmodul. Die Beweglichkeit kann vorteilhaft im Bereich von 1 bis 3 mm liegen, kann aber geringer sein, z. B. 0.4-0.8 mm. In einer weiterführenden Ausgestaltung kann die zweite Kontaktanordnung komplementär zur ,schwimmend` angeordneten ersten Kontaktanordnung starr am oder im Pumpmodul angeordnet sein. Dadurch kann beim Pumpmodul, welches über eine längere Zeit mit zahlreichen Reservoirmodulen eingesetzt wird, eine stabile und dauerhafte Anordnung gewählt werden, während bei der ersten Kontaktanordnung eine flexible Anordnung gewählt werden kann, da diese nur wenige Schliess- und Lösevorgänge des Bajonettverschlusses mitmachen muss.

In einer weiteren Ausführung umfasst das Reservoirmodul weiter einen Fluidpfad, welcher vom distalen Ende des Reservoirs zu einer Infusionskanüle führt, wobei die Infusionskanüle in das Gewebe einer benutzenden Person eingeführt sein kann, so dass beim Verdrängen von Medikamentenformulierung aus dem Reservoir, die Medikamentenformulierung vom Reservoir über den Fluidpfad in das Gewebe gelangen kann. In einer weiterführenden Ausführung umfasst das Reservoirmodul weiter eine Insertionsvorrichtung, mit welcher die Infusionskanüle in das Geweben eingebracht werden kann. Hierzu wird auf die europäischen Patentanmeldungen 18199475.7, 17209749.5, 17209764.4 sowie 17209772.7 verwiesen.

Vorteilhaft umfassen erfindungsgemässe Verbindungsstrukturen und Kontaktanordnungen Dichtungselemente, welche Reservoirmodul und/oder Pumpmodul vor dem unerwünschten Eindringen von Flüssigkeiten schützen. Hierbei kann zum Beispiel die Kontaktanordnung am Reservoirmodul eine Dichtlippe umfassen, welche die Kontakte in der Kontaktanordnung geometrisch umläuft. Beim Verbinden der Module wird die Dichtlippe zwischen Pumpmodul und Reservoirmodul zusammengepresst und dichtet so die Verbindung der Kontaktanordnungen ab. Die Dichtlippe kann aus Kunststoff, insbesondere einem elastomeren Kunststoff bestehen. Alternativ kann die Dichtlippe aus einem plastisch deformierbaren Kunststoff bestehen, welcher beim Verbinden der Module durch plastisches Fliessen den Spalt zwischen den Kontaktanordnungen dichten kann, wobei es bei dieser Variante vorteilhaft ist, wenn die Dichtlippe am Reservoirmodul angeordnet ist, da dieses typischerweise nur einmal verwendet wird. Analog können Dichtungselemente an den Verbindungsstrukturen ausgestaltet werden.

Das Pflaster gemäss einer möglichen erfindungsgemässen Verabreichungsvorrichtung, welche als Pflasterpumpe oder Patch Pumpe ausgebildet ist, ist in einer vorteilhaften Ausgestaltung am Gehäuse des Reservoirmoduls angeordnet, insbesondere fest aufgeklebt oder angeschweisst. Alternativ wäre es auch möglich das Pflaster auf einer von Reservoir- und Pumpmodul separaten Grundplatte anzuordnen, wobei dann Reservoir- und Pumpmodul in verbundenem Zustand auf der Grundplatte lösbar angebracht, insbesondere aufgeschnappt, würden.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: zeigt Pumpmodul und Reservoirmodul einer erfindungsgemässen Verabreichungsvorrichtung in getrenntem Zustand
- Figs. 2a bis 2c: zeigen den Verbindungsvorgang, bei welchem Pumpmodul und Reservoirmodul über den Bajonettverschluss verbunden werden
- Figs, 3a und 3b: Zeigen eine erfindungsgemässe Ausführung einer Verabreichungsvorrichtung
- Fig. 4: zeigt Schnittansicht durch die Verabreichungsvorrichtung in verbundenen Zustand von Pumpund Reservoirmodul aus Figs 3a und 3b durch den Bereich der Kontaktanordnungen
- Figs. 5a bis 5c: zeigen Detailansichten des Reservoirmoduls aus Figur 3b
- Fig. 6: zeigt alternative Ausführungsform des Pumpmoduls (Kontaktanordnung mit Stiften)
- Fig. 7: zeigt die alternative Ausführungsform des Pumpmoduls in einer Schnittdarstellung (Kontaktanordnung mit Buchsen)
- Figs. 8a und 8b: zeigen mögliche erfindungsgemässe Varianten der Stiftausführung für die Kontaktanordnungen
- Fig. 9: zeigt Reservoirmodul ohne Gehäuse

### FIGURENBESCHREIBUNG

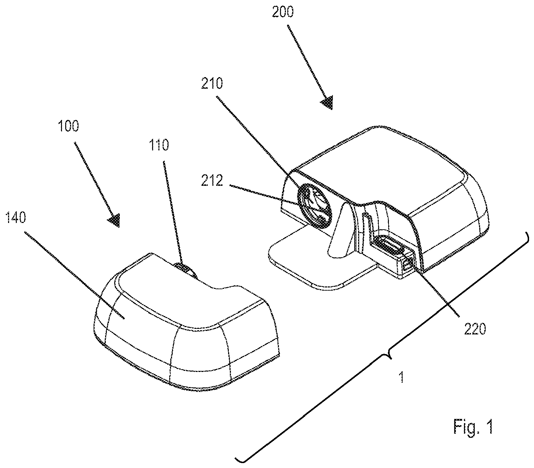

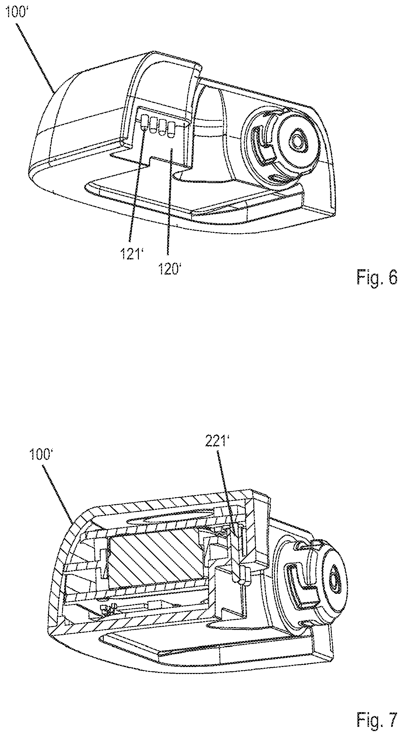

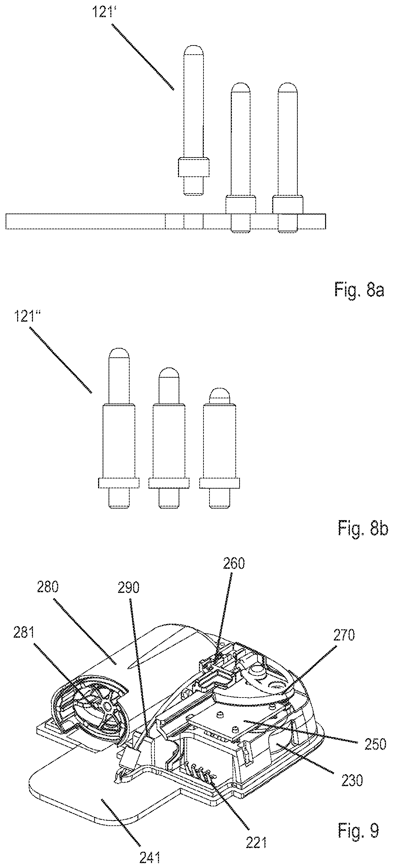

Die Figuren 1 bis 5c sowie Figur 9 zeigen eine erste Ausführungsform der Erfindung. Die Figuren 6 und 7 zeigen eine weitere erfindungsgemässe Ausführungsform. Die Figuren 8a und 8b zeigen vorteilhafte Ausgestaltungen der zweiten Ausführungsform.

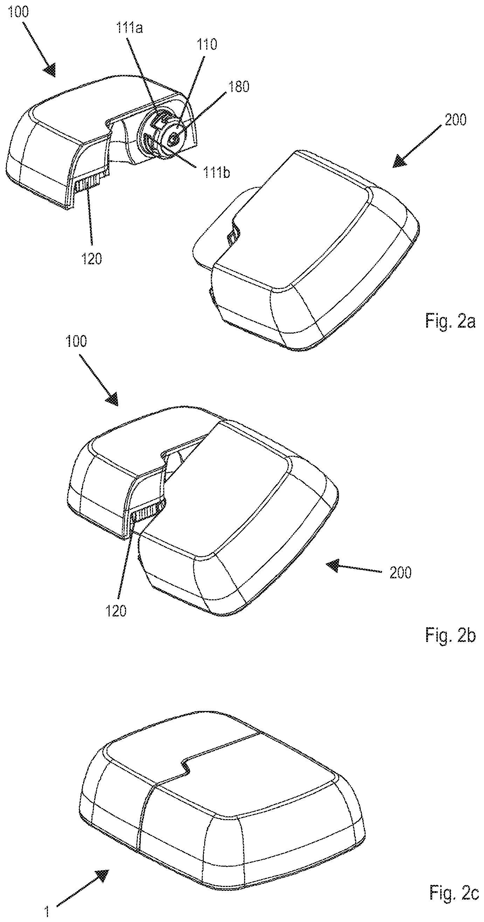

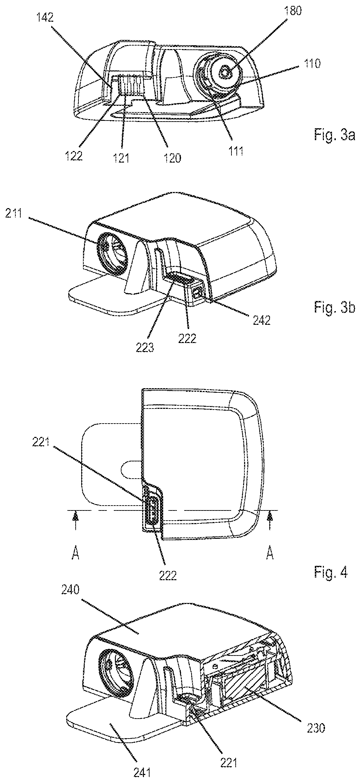

Im Folgenden wird nun Bezug auf die erste Ausführungsform genommen, welche in den Figuren 1 bis 5c und Figur 9 abgebildet ist. Das in Figur 1 dargestellte Verabreichungsgerät 1 ist eine modulare Patch Pumpe (auch Pflasterpumpe genannt), welche von der benutzenden Person direkt auf der Haut getragen werden kann. Das Gerät 1 besteht aus dem Pumpmodul 100 und dem Reservoirmodul 200. Das Pumpmodul 100 ist dabei als wiederverwendbares Modul ausgestaltet. Das Reservoirmodul 200 ist dabei beispielhaft als Einwegmodul ausgestaltet (Mehrwegmodul wäre auch möglich, ohne von der Erfindung abzuweichen). Pumpmodul 100 und Reservoirmodul 200 werden über einen Bajonettverschlusses miteinander verbunden. Hierzu umfasst das Pumpmodul 100 die Verbindungsstruktur 110, welche ihrerseits die Bajonettführungsnuten 111 umfasst, und das Reservoirmodul 200 die Verbindungsstruktur 210, welche die Führungsnocken 211 und die Öffnung 212 umfasst. Die Verbindungsstruktur 110 steht aus dem Gehäuse 140 hervor und kann in die Öffnung 212 eingeführt werden. Zum Schliessen des Bajonettverschlusses werden die beiden Module 100 und 200 zuerst so zu einander ausgerichtet, dass die Führungsnocken 211 (Figur 3b) in die Führungsnuten 111 eingeschoben werden können (Figur 2a). Dann werden die beiden Module 100 und 200 zusammen geschoben, bis dass die Führungsnocken 211 die Ecken 111a der Führungsnuten 111 erreichen (Figur 2b). In einem letzten Schritt werden die beiden Module relativ zueinander rotiert, so dass die Führungsnocken 211 in den Führungsnuten 111 in Richtung der Enden 111b bewegt werden. Zum Schluss der Bewegung verschnappen die beiden Module 100 und 200 über den Schnapperzahn 142 (Figur 3a) und die Schnappervertiefung 242 (Figur 3b), wobei die Bodenplatte des Gehäuses 140 des Pumpmoduls 100 parallel oder coplanar zur Bodenplatte 241 des Reservoirmoduls 200 zu liegen kommt (Figur 2c). Die Rotation der Module 100 und 200 relativ zueinander kann vorteilhaft von einer leichten axialen Bewegung überlagert werden, wobei die beiden Module 100 und 200 zueinander hin gezogen werden, um einen festen, spielarmen und toleranzarmen Sitz des Reservoirmoduls 200 am Pumpmodul 100 zu erzielen. Vorteilhaft an dieser Bajonettverbindung ist sodann, dass wie schon erwähnt, Toleranzen aufgehoben werden können und durch den festen Sitz des Reservoirmoduls 200 am Pumpmodul 100 ist auch eine Übertragung von Kräften, insbesondere von Kräften, welche durch den Antrieb generiert werden, zwischen den Modulen unproblematisch.

Die Verabreichungsvorrichtung 1 umfasst einen konventionellen Ausschüttmechanismus. Zum Ausschütten von Medikament kann eine im Pumpmodul angeordnete Kolbenstange 180 ins Reservoir 280 des Reservoirmoduls 200 hineingefahren werden. Der Kolben 281 des Reservoirs 280 kann dabei verschoben und damit das Volumen im Reservoir 280 verkleinert werden, wodurch Medikament aus dem Reservoir 280 verdrängt wird und durch den Infusionspfad 290 und die Infusionskanüle 291 heraus verabreicht wird (siehe Figuren 2a, 5c und 9). Die Infusionskanüle 291 kann vorgängig mittels einer Insertionskanüle 292 und einen mit dem Infusionspfad verbunden Insertionsmechanismus 260 in das Gewebe der benutzenden Person eingebracht worden sein.

Pumpmodul 100 und Reservoirmodul 200 werden beim Schliessen der Bajonettverbindung nicht nur mechanisch sondern auch elektrisch miteinander verbunden. Die geschieht über die Kontaktanordnung 120 des Pumpmoduls und die komplementär dazu ausgebildete Kontaktanordnung 220 des Reservoirmoduls 200. Erfindungsgemäss sind die Kontaktanordnungen 120 und 220 von den Verbindungsstrukturen geometrisch abgesetzt, so dass während der beschriebenen Rotation beim Schliessen der Bajonettverbindung der relative Weg, welchen die Kontaktanordnungen 120 und 220 zurücklegen, grösser ist, als derjenige, den zum Bespiel die Führungsnocken 211 zurücklegen. Die Kontaktanordnung 120 des Pumpmoduls umfasst mehrere elektrische Kontakte 121, welche streifenförmig auf einem Stecker 122 angeordnet sind (Figur 3a). Komplementär dazu umfasst die Kontaktanordnung 220 des Reservoirmoduls 200 eine Buchse 222 in welcher die Kontaktzungen 221 angeordnet sind (Figuren 3b und 4). Am Rand der Buchse 222 ist eine Dichtlippe 223 angeordnet, welche bei geschlossener Bajonettverbindung dazu dient zu verhindern, dass Flüssigkeit durch die Kontaktanordnung hindurch in das Reservoirmodul 200 eindringen kann. Die Kontaktzungen 221 sind vorzugsweise federnd ausgestaltet, zum Beispiel hergestellt aus einem metallischen Stanzbiegeteil. Beim Schliessen der Bajonettverbindung wird der Stecker 122 in die Buchse 222 eingeschoben und die Kontaktzungen 221 kontaktieren die streifenförmigen Kontakte 121 auf dem Stecker 122. Die Streifenform der Kontakte 121 ermöglicht vorteilhaft die Kontaktierung durch die Kontaktzungen 221 entlang einer gewissen Strecke. Die federnde Ausgestaltung der Kontaktzungen 221 hat weiter den Vorteil, dass sie die oben erwähnte der Rotation überlagerte axiale Bewegung aufnehmen und bei der Kontaktierung ausgleichen kann.

Das Reservoirmodul umfasst vorteilhaft, wie in den Figuren 4, 5a-c und 9 gezeigt, nebst den bereits erwähnten Elementen weitere Elemente.

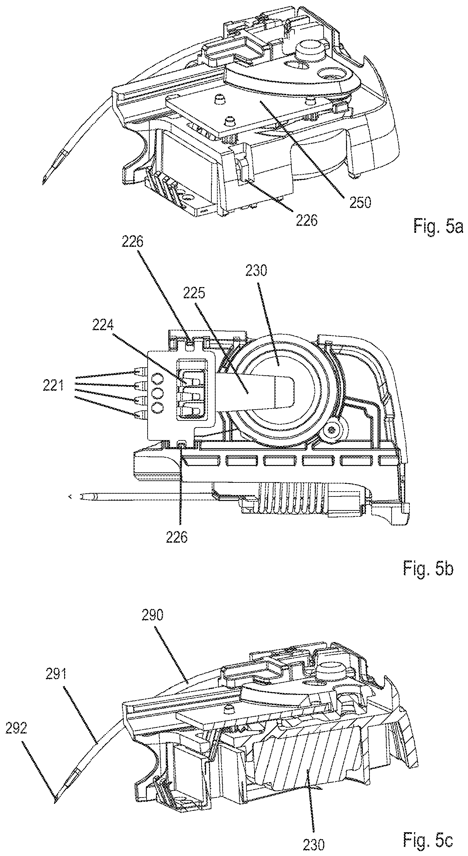

So umfasst das Reservoirmodul 200 in einer Ausgestaltung mindestens eine Energiequelle, zum Beispiel in Form der Primärzelle oder Batterie 230. Die Batterie 230 kann dazu dienen, eine im Pumpmodul angeordnete Sekundärzelle (z. B. eine wiederaufladbare Zelle) und/oder den Antrieb der Verabreichungseinrichtung mit elektrischer Energie zu versorgen. Weiter kann die Batterie dazu verwendet werden, elektrische und/oder elektronische Elemente, welche im Reservoirmodul 200 angeordnet sind, mit Energie zu versorgen. Die Figuren 5a bis 5c zeigen beispielhaft wie die Batterie 230 im Reservoirmodul angeordnet sein kann. In der gezeigten Ausführung wird die Batterie 230 durch den zungenförmigen Batteriekontakt 225 im Reservoirmodul 200 gehalten. Der Batteriekontakt 225 ist dabei in einer vorteilhaften Ausgestaltung wie die Kontaktzungen 221 federnd ausgestaltet, zum Beispiel hergestellt aus einem metallischen Stanzbiegeteil. Der Batteriekontakt 225 kontaktiert den einen Pol der Batterie 230 und ein weiterer (nicht gezeigter) Kontakt stellt die elektrische Verbindung mit dem anderen Pol der Batterie 230 her.

Weiter umfasst das Reserviormodul 200 in einer Ausgestaltung mindestens ein Elektronikboard 250 (Figuren 5a bis 5c). Das Elektronikboard 250 kann einen elektronischen Speicher zum Speichern von Information umfassen. Darin können (nicht abschliessend) Informationen wie Herstelldatum des Reservoirmoduls 200, Seriennummer, Informationen aus potentiell im Reservoirmodul vorhandene Sensoren, insbesondere ein Temperaturlogfile, oder Betriebsparameter gespeichert sein. Weiter kann das Elektronikboard 250 eine Steuerung für den weiter oben erwähnten Insertionsmechanismus 260 (Figur 9) umfassen. In einer vorteilhaften Ausgestaltung wird dabei von der Steuerung eine Auslöseanordnung 270 für den Insertionsmechanismus 260 gesteuert. Das Elektronikboard kann beispielhaft über die Kontakte 224 (insbesondere Figuren 5b und 5c) mit der Batterie 230 oder der Kontaktanordnung 220 verbunden sein. In einer vorteilhaften Ausführung werden die Kontaktzungen 221, die Kontakte 224 sowie der Batteriekontakt 225 aus einem Stanzbiege- /Kunststoffhybridbauteil hergestellt, insbesondere aus einem Bauteil bestehend aus einem metallischen Stanzbiegeteil umspritzt mit einem elektrisch isolierend wirkenden Kunststoff. Unter anderem vorteilhaft an dieser Ausführung ist, dass das Stanzbiege-Kunststoff-Hybridbauteil sodann in einem Montageschritt zusammen mit der Batterie 230 am Reservoirmodul montiert, insbesondere verschnappt (siehe Element 226 in Figuren 5a und 5b) werden kann.

Weiter umfasst das Reservoirmodul 200 in einer Ausgestaltung die schon erwähnte Auslöseanordnung 270 für den Insertionsmechanismus 260, wobei für mögliche Ausführungen der Anordnung 270 und des Mechanismus 260 an dieser Stelle noch einmal auf die europäischen Patentanmeldungen 18199475.7, 17209749.5, 17209764.4 sowie 17209772.7 verwiesen wird.

Die Figuren 6 und 7 zeigen eine zweite erfindungsgemässe Ausführungsform. Die Figuren 8a und 8b zwei mögliche Ausgestaltungsdetails der zweiten Ausführungsform. Im Folgenden wird auf die Unterschiede zur ersten Ausführungsform eingegangen. Die anderen Aspekte stimmen mit der ersten Ausführungsform weitestgehend überein und werden per Verweis auch in diese Ausführungsform übernommen. Der Hauptunterschied zur ersten Ausführungsform liegt in den Kontaktanordnungen von Pumpmodul 100' und dem angepassten Reservoirmodul (nicht gezeigt). Die Kontaktanordnung 120' umfasst nicht einen einzelnen Stecker, auf welchem Kontakte angeordnet sind, sondern einzelne Kontaktstifte 121'. Das Reservoirmodul (nicht gezeigt) kann komplementär dazu einzelne Buchsen für jeden Kontaktstift umfassen. Hierbei sind die einzelnen Buchsen in einer vorteilhaften Ausgestaltung schwimmend im Reservoirmodul (nicht gezeigt) gelagert, so dass eine allfällige axiale Bewegung während der erwähnten Rotationsbewegung beim Schliessen des Bajonetts ausgeglichen werden kann. In einer alternativen Ausgestaltung kann das Reservoirmodul für die zweite Ausführungsform auch ähnlich oder sogar gleich wie in der ersten Ausführungsform ausgestaltet, so dass nur eine Buchse alle Kontaktstifte aufnimmt. Hierbei können die Kontaktzungen in der Kontaktanordnung des Reservoirmoduls (nicht gezeigt) geometrisch so geformt werden, dass sie gut zu den Stiften passen.

Die Figuren 8a und 8b zeigen zwei alternative Ausgestaltungen der Kontaktstifte. In Figur 8a weisen die Stifte 121' jeweils eine feste Länge auf. Figur 8b zeigt eine Alternative, bei welcher die Stifte als federnde und teleskopierbare Stifte 121 "mit variabler Länge ausgestaltet sind, zum Beispiel als sogenannte Pogo-Stifte. Kommt diese alternative Ausgestaltung der Kontaktstifte 121" zum Einsatz, kann im Gegenzug die Kontaktanordnung im Reservoirmodul (nicht gezeigt) vereinfacht werden. Die Kontaktanordnung des Reservoirmoduls (nicht gezeigt) kann sodann einfacher und unbeweglich am Reservoirmodul (nicht gezeigt) angeordnete Kontaktflächen aufweisen.

### BEZUGSZEICHENLISTE

- 1: Verabreichungsvorrichtung
- 100: Pumpmodul
- 100': Pumpmodul
- 110: Verbindungstruktur Pumpmodul
- 111: Führungsnuten Bajonett
- 111a: Ecke
- 111b: Ende Führungsnut
- 120: Kontaktanordnung Pumpmodul
- 120': Kontaktanordnung Pumpmodul
- 121: elektrische Kontakte
- 121': elektrische Kontaktstifte
- 121": teleskopierbare elektrische Kontaktstifte
- 122: Stecker
- 140: Gehäuse Pumpmodul
- 142: Schnapperzahn
- 180: Kolbenstange Pumpmodul

- 200: Reservoirmodul
- 210: Verbindungstruktur Reservoirmodul
- 211: Führungsnocken Bajonett
- 212: Öffnung
- 220: Kontaktanordnung Reservoirmodul
- 221: elektrische Kontaktzungen
- 222: Buchse
- 223: Dichtlippe
- 224: Kontaktierung Elektronikboard
- 225: Batteriekontakt
- 226: Verschnappung
- 230: Primärzelle oder Batterie
- 240: Gehäuse Reservoirmodul
- 241: Bodenplatte Reservoirmodul
- 242: Schnappervertiefung
- 250: Elektronikboard Reservoirmodul
- 260: Insertionsmechanismus für Infusionskanüle
- 270: Auslöseanordnung für Insertionsmechanismus
- 280: Reservoir
- 281: Reservoirkolben
- 290: Infusionspfad
- 291: Infusionskanüle
- 292: Insertionskanüle/-nadel

## Patentansprüche

1. Modulare Vorrichtung (1) zur Verabreichung einer fluiden Medikamentenformulierung, welche auf der Haut einer benutzenden Person fixierbar ist, umfassend
ein Reservoirmodul (200) mit einem Reservoir (280) für die Medikamentenformulierung, wobei das Reservoir (280) eine erste Achse definiert, und wobei das Reservoirmodul (200) eine erste Verbindungstruktur (210) sowie eine erste elektrische Kontaktanordnung (220) umfasst, und
ein Pumpmodul (100) mit einer Kolbenstange (180), welche eine zweite Achse definiert, wobei das Pumpmodul (100) eine zweite Verbindungsstruktur (110) umfasst, welche um die Kolbenstange (180) herum angeordnet ist, und wobei das Pumpmodul (100) weiter eine zweite elektrische Kontaktanordnung (120) umfasst,
wobei das Reservoirmodul (200) und das Pumpmodul (100) lösbar über die erste und die zweite Verbindungstruktur (110) miteinander verbindbar sind, so dass durch Bewegung der Kolbenstange (180) Medikamentenformulierung aus dem Reservoir (280) verdrängbar ist,
wobei zum Verbinden der Module (100, 200)
in einem ersten Schritt die erste und die zweite Verbindungsstruktur (210, 110) zusammengesteckt werden, so dass die erste und zweite Achse auf eine gemeinsame Achse zu liegen kommen, und so eine Rotationsachse bilden,
in einem zweiten Schritt das Pumpmodul (100) relativ zum Reservoirmodul (200) um die Rotationsachse rotiert wird, und beim zweiten Schritt durch die Rotation die erste Kontaktanordnung (220) mit der zweiten Kontaktanordnung (120) elektrisch leitend verbunden wird,
wobei die erste Kontaktanordnung (220) in radialer Richtung von der ersten Achse weiter entfernt ist als die erste Verbindungsstruktur (210), und
die zweite Kontaktanordnung (120) in radialer Richtung von der zweiten Achse weiter entfernt ist als die zweite Verbindungsstruktur (110).

2. Modulare Vorrichtung nach Anspruch 1, **dadurch gekennzeichnet, dass** die erste und die zweite Verbindungsstruktur (210, 110) zusammen einen Bajonettverschluss bilden.

3. Modulare Vorrichtung nach Anspruch 1 oder 2, wobei die erste Kontaktanordnung (220) mindestens eine Buchse (222) umfasst und die zweite Kontaktanordnung (120) komplementär mindestens einen in die Buchse (222) passenden Stift (121) oder Stecker (122) umfasst.

4. Modulare Vorrichtung nach Anspruch 1 oder 2, wobei die erste Kontaktanordnung (220) mehrere parallel zueinander angeordnete Buchsen (222) umfasst und die zweite Kontaktanordnung (120) passend eine entsprechende Anzahl Stifte (121) oder Stecker (122) umfasst, welche geometrisch in die Buchsen (222) passen.

5. Modulare Vorrichtung nach Anspruch 3 oder 4, wobei die Buchsen (222) der ersten Kontaktanordnung (220), sowie die Stifte (121) oder Stecker (122) der zweiten Kontakanordnung in etwa senkrecht zur ersten respektive zweiten Achse stehen.

6. Modulare Vorrichtung nach einem der vorhergehenden Ansprüche, wobei die erste Kontaktanordnung (220) beweglich, insbesondere schwimmend, im Reservoirmodul (200) gelagert ist.

7. Modulare Vorrichtung nach Anspruch 6, wobei die zweite Kontaktanordnung (120) starr am Pumpenmodul (100) angeordnet ist.

8. Modulare Vorrichtung nach einem der vorhergehenden Ansprüche, wobei das Reservoirmodul (200) weiter einen Fluidpfad (290) mit einer Infusionskanüle (291) und einen Insertionsmechanismus (260) zum Einbringen der Infusionskanüle (291) in das Gewebe der benutzenden Person umfasst.

9. Modulare Vorrichtung nach einem der vorhergehenden Ansprüche, wobei das Reservoirmodul (200) weiter einen Speicher für elektrische Energie, insbesondere eine Primärzelle oder Batterie (230), und einen zungenförmigen Batteriekontakt (225) umfasst, wobei der Batteriekontakt (225) ein freies federndes Ende und ein am Reservoirmodul fest angeordnetes Ende umfasst, und wobei die das freie Ende den Speicher für elektrische Energie (230) in ein vorgesehenes Volumen im Reservoir-modul (200) hineindrückt und hält.

## Claims

1. Modular device (1) for administering a fluid medicament formulation, which can be fixed to the skin of a user, comprising
a reservoir module (200) having a reservoir (280) for the medicament formulation, wherein the reservoir (280) defines a first axis, and the reservoir module (200) comprises a first connection structure (210) and a first electrical contact arrangement (220), and
a pump module (100) having a piston rod (180) defining a second axis, wherein the pump module (100) comprises a second connection structure (110) arranged around the piston rod (180), and wherein the pump module (100) further comprises a second electrical contact arrangement (120),
wherein the reservoir module (200) and the pump module (100) are detachably connectable to one another via the first and second connecting structures (110), so that medicament formulation can be displaced from the reservoir (280) by movement of the piston rod (180),
wherein the first and second connecting structures (210, 110) are plugged together in a first step for connecting the modules (100, 200), so that the first and second axes lie on a common axis and thus form an axis of rotation,
the pump module (100) is rotated relative to the reservoir module (200) about the axis of rotation in a second step, and the first contact arrangement (220) is electrically connected to the second contact arrangement (120) by the rotation during the second step,
wherein the first contact arrangement (220) is further away from the first axis in the radial direction than the first connection structure (210), and
the second contact arrangement (120) is further away from the second axis in the radial direction than the second connection structure (110).

2. Modular device according to claim 1, **characterized in that** the first and the second connecting structure (210, 110) together form a bayonet closure.

3. Modular device according to either claim 1 or claim 2, wherein the first contact arrangement (220) comprises at least one socket (222) and the second contact arrangement (120) complementarily comprises at least one pin (121) or plug (122) which fits into the socket (222).

4. Modular device according to either claim 1 or claim 2, wherein the first contact arrangement (220) comprises a plurality of sockets (222) arranged in parallel with one another, and the second contact arrangement (120) comprises a corresponding number of pins (121) or plugs (122) which fit geometrically into the sockets (222).

5. Modular device according to either claim 3 or claim 4, wherein the sockets (222) of the first contact arrangement (220), as well as the pins (121) or plugs (122) of the second contact arrangement, are approximately perpendicular to the first or second axis, respectively.

6. Modular device according to any of the preceding claims, wherein the first contact arrangement (220) is mounted movably, in particular floatingly, in the reservoir module (200).

7. Modular device according to claim 6, wherein the second contact arrangement (120) is rigidly arranged on the pump module (100).

8. Modular device according to any preceding claim, wherein the reservoir module (200) further comprises a fluid path (290) having an infusion cannula (291) and an insertion mechanism (260) for inserting the infusion cannula (291) into the tissue of the user.

9. Modular device according to any of the preceding claims, wherein the reservoir module (200) further comprises a storage device for electrical energy, in particular a primary cell or battery (230), and a tongue-shaped battery contact (225), wherein the battery contact (225) comprises a free resilient end and an end fixedly arranged on the reservoir module, and wherein the free end presses the storage device for electrical energy (230) into a provided volume in the reservoir module (200) and holds it there.

## Revendications

1. Dispositif modulaire (1) pour l'administration d'une formulation médicamenteuse fluide, lequel peut être fixé sur la peau d'une personne utilisatrice, comprenant
un module pour réservoir (200) comportant un réservoir (280) pour la formulation médicamenteuse, dans lequel le réservoir (280) définit un premier axe, et dans lequel le module pour réservoir (200) comprend une première structure de liaison (210) ainsi qu'un premier agencement de contact (220) électrique, et
un module de pompage (100) comportant une tige de piston (180) qui définit un second axe, dans lequel le module de pompage (100) comprend une seconde structure de liaison (110) qui est disposée autour de la tige de piston (180), et dans lequel le module de pompage (100) comprend en outre un second agencement de contact (120) électrique,
dans lequel le module pour réservoir (200) et le module de pompage (100) peuvent être reliés l'un à l'autre de manière amovible par l'intermédiaire de la première et de la seconde structure de liaison (110), de sorte que la formulation médicamenteuse peut être déplacée hors du réservoir (280) par le mouvement de la tige de piston (180),
dans lequel, pour la liaison des modules (100, 200),
dans une première étape, la première et la seconde structure de liaison (210, 110) sont emboîtées l'une dans l'autre de sorte que le premier et le second axe viennent se placer sur un axe commun, formant ainsi un axe de rotation,
dans une seconde étape, le module de pompage (100) est mis en rotation autour de l'axe de rotation par rapport au module pour réservoir (200) et, lors de la seconde étape, le premier agencement de contact (220) est connecté de manière électriquement conductrice au second agencement de contact (120) par la rotation,
dans lequel le premier agencement de contact (220) est plus éloigné du premier axe dans une direction radiale que la première structure de liaison (210), et
le second agencement de contact (120) est plus éloigné du second axe dans une direction radiale que la seconde structure de liaison (110).

2. Dispositif modulaire selon la revendication 1, **caractérisé en ce que** la première et la seconde structure de liaison (210, 110) forment conjointement une fermeture à baïonnette.

3. Dispositif modulaire selon la revendication 1 ou 2, dans lequel le premier agencement de contact (220) comprend au moins une douille (222) et le second agencement de contact (120) comprend de manière complémentaire au moins une broche (121) ou une fiche (122) s'adaptant dans la douille (222).

4. Dispositif modulaire selon la revendication 1 ou 2, dans lequel le premier agencement de contact (220) comprend plusieurs douilles (222) disposées parallèlement les unes aux autres et le second agencement de contact (120) comprend, de manière adaptée, un nombre correspondant de broches (121) ou de fiches (122) qui s'adaptent géométriquement dans les douilles (222).

5. Dispositif modulaire selon la revendication 3 ou 4, dans lequel les douilles (222) du premier agencement de contact (220), ainsi que les broches (121) ou les fiches (122) du second agencement de contact, sont approximativement perpendiculaires aux premier et second axes, respectivement.

6. Dispositif modulaire selon l'une des revendications précédentes, dans lequel le premier agencement de contact (220) est monté mobile, en particulier flottant, dans le module pour réservoir (200).

7. Dispositif modulaire selon la revendication 6, dans lequel le second agencement de contact (120) est disposé de manière rigide sur le module de pompage (100).

8. Dispositif modulaire selon l'une des revendications précédentes, dans lequel le module pour réservoir (200) comprend en outre un trajet de fluide (290) comportant une canule de perfusion (291) et un mécanisme d'insertion (260) pour l'introduction de la canule de perfusion (291) dans le tissu de la personne utilisatrice.

9. Dispositif modulaire selon l'une des revendications précédentes, dans lequel le module pour réservoir (200) comprend en outre un accumulateur d'énergie électrique (230), en particulier une pile primaire ou une batterie, et un contact de batterie (225) en forme de languette, dans lequel le contact de batterie (225) comprend une extrémité libre élastique et une extrémité disposée de manière fixe sur le module pour réservoir, et dans lequel l'extrémité libre pousse et maintient l'accumulateur d'énergie électrique (230) dans un volume prévu dans le module pour réservoir (200).
